# EUROPÄISCHE PATENTSCHRIFT

(11) **EP 0 626 368 B1**
(45) Veröffentlichungstag und Bekanntmachung des Hinweises auf die Patenterteilung: **15.04.1998**
(21) Anmeldenummer: 94107545.9
(22) Anmeldetag: 16.05.1994
(51) Int. Cl.: C07C 263/20, C07C 263/10

(54) **Verfahren zur Herstellung von Isocyanaten und kontinuierlicher Aufarbeitung des Rückstandes**
Process for preparing isocyanates and continuous treatment of the residue
Procédé de préparation d'isocyanates et traitement en continu de résidu

(30) Priorität: 27.05.1993 DE 4317669
(43) Veröffentlichungstag der Anmeldung: 30.11.1994
(73) Patentinhaber: BAYER AG, 51368 Leverkusen (DE)
(72) Erfinder: Benedix, Hermann-Josef, D-25709 Marne (DE); Motika, Werner Dipl.-Ing., D-25709 Marne (DE)

(56) Entgegenhaltungen:
- DE-A- 2 012 294
- US-A- 2 889 257
- US-A- 3 457 291
- US-A- 4 216 063

## Beschreibung

Die Erfindung betrifft ein Verfahren zur Herstellung von reinen, destillierten Isocyanaten durch Umsetzung der entsprechenden Amine mit Phosgen in einem geeigneten Lösungsmittel und mehrstufige destillative Aufarbeitung in reine Isocyanate, reines Lösungsmittel und einen Anteil an Rückstand, wobei dieser Rückstand kontinuierlich aufgearbeitet wird.

Die großtechnische Herstellung destillierter Isocyanate durch Umsetzung von Aminen mit Phosgen in Lösungsmitteln ist bekannt und in der Literatur ausführlich beschrieben (Ullmanns Encyklopädie der technischen Chemie, 4. Auflage, Band 13, Seite 347-357, Verlag Chemie, GmbH, D-6940 Weinheim, 1977). Wie in dieser Literatur weiterhin beschrieben. ist, fällt bei der Herstellung reiner destillierter Isocyanate in den Destillationsprozessen ein Nebenproduktstrom an, der nach weitmöglicher destillativer Abtrennung von freien Isocyanaten als Rückstand entsorgt werden muß. Im Laborversuch ist es möglich, aus diesem Rückstandsstrom erhebliche Anteile an freiem Isocyanat weiter abzudestillieren. Allerdings wird der verbleibende Rückstand zu einer harten vernetzten Masse, die im technischen Prozeß nicht handhabbar ist. Im technischen Prozeß ist man daher gezwungen ca. 20-40 % freies Isocyanat in dem zu entsorgenden Rückstandsstrom zu belassen, damit dieser Rückstandsstrom technisch handhabbar bleibt. Dies hat unter anderem den erheblichen Nachteil, daß einerseits Wertstoff verloren geht und andererseits mehr Abfall entsorgt werden muß.

Es sind deshalb Prozesse bekannt, um weiteres freies Isocyanat aus dem Rückstand zu gewinnen. Z.B. beschreibt das britische Patent 1 408 745 einen Extraktionsprozeß zur Gewinnung des freien Isocyanates. In EP 269 218 wird ein Aufarbeitungsprozeß durch Erhitzen dieses Rücksrandsstroms mit einem Bad von geschmolzenem Metall oder Metallsalzen beschrieben. Aus DE 2 915 830 geht ein Destillationsprozeß dieses Rückstandsstroms in einem Fließbett hervor. All diese Prozesse erfordern aufwendige Apparate und/oder Hilfsstoffe, wobei Hilfsstoffe wie z.B. Meile oder Metallsalze die spätere Entsorgung erheblich erschweren.

In US-A 4,216,063 wird ein kontinuierliches Verfahren zur destillativen Aufarbeitung eines Rückstandes mit Hilfe der Verwendung eines produktumschaufelnden Vakuumtrockners beschrieben. Das dort beschriebene Verfahren ist jedoch wie die anderen beschriebenen Verfahren mit den beschriebenen Nachteilen ebenfalls behaftet.

Aufgabe war es daher, ein technisch einfaches und sicheres Verfahren zu entwickeln, das es erlaubt, weiteres Isocyanat aus den Rückstanden des Destillationsprozesses der Isocyanatproduktion zu gewinnen, ohne daß die oben genannten Nachteile die Aufarbeitung ökologisch und ökonomisch verkomplizieren, und zwar so, daß der verbleibende nicht weiter aufarbeitbare Rückstand einfach und ohne Belastung der Umwelt zu entsorgen ist.

Diese Aufgabe konnte überraschenderweise mit dem erfindungsgemäßen Verfahren gelöst werden.

Gegenstand der Erfindung ist ein Verfahren zur Herstellung von reinen, destillierten Isocyanaten durch Umsetzung der entsprechenden Amine mit Phosgen in einem geeigneten Lösungsmittel und mehrstufige destillative Aufarbeitung der erhaltenen Isocyanatlösung in reine Isocyanate, reines Lösungsmittel und einen Anteil an Rückstand, welches dadurch gekennzeichnet ist, daß der aus dem Destillationsprozeß erhaltene Rückstand kontinuierlich zusammen mit 2 bis 50 Gew.-% hochsiedenden Kohlenwasserstoffen, vorzugsweise Bitumen, die unter den Destillationsbedingungen inert sind, einem beheizten, produktumschaufelnden Vakuumtrockner mit horizontaler Welle zugeführt wird, der Anteil an noch vorhandenen Isocyanaten bei Temperaturen von 160 bis 280°C und Drücken von 2 bis 50 mbar kontinuierlich aus dem Rückstand abdestilliert wird und der verbleibende Rückstand als rieselfähiges, nicht staubendes Granulat kontinuierlich ausgetragen, gegebenenfalls abgekühlt und gegebenenfalls nach Mahlung verbrannt wird.

Überraschenderweise konnte mit den hochsiedenden Kohlenwasserstoffen oder Kohlenwasserstoffgemischen, vorzugsweise Bitumen, unter den entsprechenden Destillationsbedingungen in Abhängigkeit von den Isocyanaten weiteres Isocyanat aus dem Rückstand abdestilliert werden. Dabei entstand unter den Reaktionsbedingungen aus dem Gemisch an hochsiedenden Kohlenwasserstoffen und nicht destillierbarem Polymeranteil des Rückstandes eine krümelige, freifließende Masse, die praktisch frei an Isocyanat ist. Vor einer Zwischenlagerung oder Weiterverarbeitung wird der Rückstand abgekühlt.

Ein weiterer Vorteil des erfindungsgemäßen Verfahrens besteht darin, daß bei der Aufarbeitung des Rückstandes vorhandene chlorhaltige Verunreinigungen größtenteils entfernt werden und diese wieder verwendet werden können

Das erfindungsgemäße Verfahren kann allgemein für die Herstellung verschiedener Isocyanate eingesetzt werden. Insbesondere werden mit diesem Verfahren folgende Isocyanate hergestellt: Toluylendiisocyanat, 1,6-Hexandiisocyanat, 1-Isocyanato-3,3,5-Trimethyl-5-Isocyanatomethylcyclohexan, 1,5-Naphthylendiisocyanat, Diisocyanatodiphenylmethan.

Der Rückstand, wie er bei der destillativen Aufarbeitung bei der Herstellung von Isocyanaten anfällt, enthält normalerweise 20-80 Gew.-% freies Isocyanat neben den polymeren Nebenprodukten, bevorzugt 40-60 Gew.-% freies Isocyanat.

Über dem abgekühlten, ausgetragenen, verbliebenen Rückstand nach Durchführung des erfindungsgemäßen Verfahrens sind keine physiologisch wirksamen Dampfanteile an Isocyanat mehr nachweisbar, da praktisch fast alles Isocyanat abdestilliert werden konnte.

Der Rückstand kann vorzugsweise getrennt von den Kohlenwasserstoffen in mehreren Teilströmen dem Trockner zugeführt werden.

Besonders bevorzugt wird ein Teil des Rückstandes mit den Kohlenwasserstoffen gemischt und dem Trockner zugeführt und der restliche Teil des Rückstandes in einem oder mehreren Teilströmen dem Trockner zugeführt.

Der ausgetragene, körnige Rückstand wird vorzugsweise vor der Entsorgung, z.B. Verbrennung, abgekühlt

Ein weiterer Gegenstand der Erfindung ist die Verwendung einer Vorrichtung aus einem kontinuierlich arbeitenden, beheizten, produktschaufelnden Vakuumtrockner mit horizontaler Welle und mehreren, über die Trocknerlänge verteilten Produktzuführungen zum Entfernen von Isocyanaten aus Rückständen aus der Destillation von Isocyanaten.

Als Trockner kann ein normaler, kontinuierlich arbeitender Kontakttrockner mit einem Doppelmantel zur Beheizung und einer horizontalen, produktumschaufelnden, ebenfalls beheizten Welle eingesetzt werden. Er hat mehrere Stutzen für den Produkteintritt, einen Stutzen für den Produktaustritt und großdimensionierte Brüdenaustrittsstutzen für das abdestillierte Isocyanat und Lösungsmittel sowie sonstige Hilfseinrichtungen. Bevorzugt werden Apparate mit Einrichtungen zur Abreinigung sowohl der Welle wie auch des Trocknergehäuses verwendet. Eine besonders bevorzugte Ausführungsform der Abreinigungseinrichtung für die Welle sind am Gehäuse befestigte Gegenhaken. Es können sowohl Einwellentrockner als auch Zweiwellentrockner oder Schneckenapparaturen eingesetzt werden.

Anfallendes Kondensat im angeschlossenen Brüdensystem wird zur Entfernung von gegebenenfalls auftretenden Staubablagerungen an den Wänden derselben getrennt abgeführt.

Der Reaktor wird bei einer Temperatur von 160-280°C, bevorzugt 200-250°C, unter einem Druck von 2-50 mbar, bevorzugt 10-20 mbar, bei einem Durchsatz von bis zu 250 kg/h pro m² Heizfläche betrieben. Vorzugsweise erfolgt die kontinuierliche Destillation in einem produktumschaufelnden Trockner mit horizontaler Welle, an den sich ein Kondensationssystem anschließt. Die Destillation erfolgt in Gegenwart von Kohlenwasserstoffen, die in einer Menge von 2-50 Gew.%, bevorzugt 10-20 Gew.-%, bezogen auf den Rückstand, zugemischt werden. Als Kohlenwasserstoffe sind reine Kohlenwasserstoffe oder auch technische Gemische, vorzugsweise Bitumen, mit einer Siedepunktdifferenz zum Isocyanat bei 15 mbar von mindestens 150°C einsetzbar. Besonders bevorzugt, vor allem unter wirtschaftlichen Gesichtspunkten, werden Asphalte und Bitumen, wie sie als Nebenprodukte in der Erdölraffinerie technisch anfallen, eingesetzt.

Ganz besonders bevorzugt werden Bitumen, z.B. des Typs B 80, B 80 E, des Typs B 300 oder B 300 E eingesetzt (Kennzeichnung nach DIN 52 010).

Der über eine Vakuumschleuse ausgetragene Rückstand kann kontinuierlich über die gekühlten Flächen z.B. einer Förderschnecke abgekühlt werden.

Die Erfindung soll anhand der nachfolgenden Beispiele näher erläutert werden.

### Beispiel 1

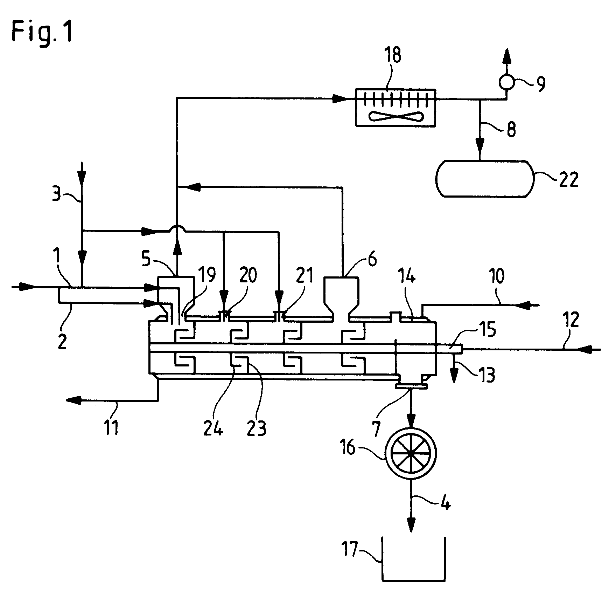

Man verwendet eine Apparatur nach Abbildung 1, bestehend aus einem liegenden zylindrischen Gehäuse (14) mit einem Innendurchmesser von 280 mm und einer Länge von 1 700 mm mit 3 Produkteinlaufstutzen (19), (20), (21), einem Produktaustragsstutzen (7) und zwei Gasaustrittsstutzen (5), (6). Die Produktumschaufelung und der axiale Produkttransport erfolgt durch eine mit Mischbarren (24) versehene Welle (15). Durch Gegenhaken (23), die am Gehäuse (14) befestigt sind, wird die Welle von Produktanbackungen weitgehend freigehalten.

Über die Eintrittsstutzen (10), (12) und die Austrittsstutzen (11), (13) wird das Gehäuse (14) und die Welle (15) mit Wärmeträgeröl beheizt.

Die Produktzufuhr erfolgt in die Eintragsstutzen (19), (20), (21). Am Produktaustragsstutzen (7) angebracht ist eine vakuumdichte Zellenradschleuse (16), die das Produkt in einen Auffangbehälter (17) fördert. Die Brüdenstutzen (5), (6) sind mit einem luftgekühlten Kondensator (18) verbunden, der an eine Vakuumpumpe (9) angeschlossen ist. Das anfallende Kondensat (8) wird einem Behälter (22) zugeleitet.

Für den Versuch wird aus der technischen Produktion von Toluylendiisocyanat (2,4-/2,6-Isomerengemiswch mit 80 % 2,4-Toluylendiisocyanat) ein Produkt der Zusammensetzung 67,4 Gew.-% 2,4- und 2,6-Toluylendiisocyanat, 29,1 Gew.-% polymerer Rückstand, 3,5 Gew.-% Lösungsmittel entnommen.

In den auf eine Innentemperatur von 240°C erhitzten und auf einen Druck von 12 mbar evakuierten Kontakttrockner werden 57 kg/h (3) des oben beschriebenen Produktes, dem 8,3 kg/h Bitumen des Typs B 80 (1) zugemischt werden, am Eintrittsstutzen (19) kontinuierlich mit einer Temperatur von 140°C eingefahren. Durch die drehende Welle (15) wird dem einlaufenden Produktstrom über die beheizten Flächen von Welle und Gehäuse Wärme zugeführt. Das im Zulauf enthaltende Toluylendiisocyanat und Lösungsmittel wird ausgedampft, dem Luftkühler (18) zugeleitet, und man erhält einen Destillatanfall von 40,2 kg/h (8), bestehend aus 38,2 kg Toluylendiisocyanat und 2,0 kg Lösungsmittel. Ausgetragen über die Zellenradschleuse (16) werden 25,1 kg/h (4) eines rieselfähigen, geruchsfreien, nicht staubenden Granulates mit einem nicht destillierbaren Resttoluylendiisocyanatgehalt von < 1 %.

### Beispiel 2

Beispiel 1 wird bezüglich der eingefahrenen Produktmenge und Produktzusammensetzung wiederholt, mit dem Unterschied, daß die Bitumenmenge des Typs B 80 von 8,3 kg/h auf 3,0 kg/h reduziert wird und die Produktmenge auf 3 Eintrittsstutzen (19), (20), (21) verteilt wird. Dabei werden 10,3 kg/h Produkt, dem 3,0 kg/h Bitumen zugemischt werden, in den Eintrittsstutzen (19) eingefahren. Die restliche Produktmenge von 46,7 kg/h wird je zur Hälfte in die Eintrittsstutzen (20), (21) eingefahren.

Die Menge und Zusammensetzung des anfallenden Destillates und die Eigenschaften des erhaltenen Rückstandes sind mit denen aus Beispiel 1 vergleichbar.

### Beispiel 3

Beispiel 2 wird bezüglich der eingefahrenen Produktmenge, Produktzusammensetzung und Bitumenmenge des Typs B 80 wiederholt, mit dem Unterschied, daß die Bitumenmenge (2) auf 230°C aufgeheizt wird und separat in den Eintrittsstutzen (19) eingefahren wird.

Die Menge zund Zusammensetzung des anfallenden Destillates und die Eigenschaften des erhaltenen Rückstandes sind mit denen aus Beispiel 1 vergleichbar.

## Patentansprüche

1. Verfahren zur Herstellung von reinen, destillierten Isocyanaten durch Umsetzung der entsprechenden Amine mit Phosgen in einem geeigneten Lösungsmittel und mehrstufige destillative Aufarbeitung der erhaltenen Isocyanatlösung in reine Isocyanate, reines Lösungsmittel sowie in einen Anteil an Rückstand, dadurch gekennzeichnet, daß der aus dem Destillationsprozeß erhaltene Rückstand kontinuierlich zusammen mit 2-50 Gew.-% hochsiedenden Kohlenwasserstoffen, vorzugsweise Bitumen, die unter den Destillationsbedingungen inert sind, einem beheizten, produktumschaufelnden Vakuumtrockner mit horizontaler Welle zugeführt wird, der Anteil an noch im Rückstand vorhandenen Isocyanaten bei Temperaturen von 160 bis 280°C und Drücken von 2 bis 50 mbar kontinuierlich abdestilliert wird und der verbleibende Rückstand als rieselfähiges, nicht staubendes Granulat kontinuierlich ausgetragen, gegebenenfalls abgekühlt und gegebenenfalls nach Mahlung einer Verbrennung zugeführt wird.

2. Verfahren nach Anspruch 1, dadurch gekennzeichnet, daß der Rückstand getrennt von den Kohlenwasserstoffen in mehreren Teilströmen dem Trockner zugeführt wird.

3. Verfahren nach Anspruch 1, dadurch gekennzeichnet, daß ein Teil des Rückstandes gemischt mit den Kohlenwasserstoffen dem Trockner zugeführt und der restliche Rückstand in einem oder mehreren Teilströmen dem Trockner zugeführt wird.

4. Verwendung einer Vorrichtung aus einem kontinuierlich arbeitenden, beheizten, produktumschaufelnden Vakuumtrockner mit horizontaler Welle und mehreren, über die Trocknerlänge verteilten Produktzuführungen zum Entfernen von Isocyanaten aus Rückständen aus der Destillation von Isocyanaten.

## Claims

1. A process for the preparation of pure, distilled isocyanates by reaction of the corresponding amines with phosgene in a suitable solvent and multistep working up, by means of distillation, of the resulting isocyanate solution into pure isocyanates, pure solvent and a portion of residue, characterised in that the residue obtained from the distillation process is passed continuously together with 2 to 50 wt.% of high-boiling hydrocarbons, preferably bitumen, which are inert under the distillation conditions, to a heated vacuum dryer having a horizontal shaft where the product is agitated, the portion of isocyanates still present in the residue is distilled off continuously at temperatures of from 160°C to 280°C and at pressures of from 2 to 50 mbar and the remaining residue is discharged continuously as flowing, dust-free granular material, is optionally cooled and removed for combustion after optional grinding.

2. A process according to claim 1, characterised in that the residue separated by the hydrocarbons is passed in several split streams to the dryer.

3. A process according to claim 1, characterised in that part of the residue mixed with the hydrocarbons is passed to the dryer and the remaining residue is passed in one or more split streams to the dryer.

4. The use of a device comprising a continuously operating, heated vacuum dryer having a horizontal shaft where the product is agitated and several product inlets distributed along the length of the dryer for the removal of the isocyanates from residues from the distillation of isocyanates.

## Revendications

1. Procédé de fabrication d'isocyanates purs distillés par conversion de l'amine correspondante avec du phosgène dans un solvant approprié et traitement par distillation en plusieurs étapes de la solution d'isocyanate résultante, pour obtenir de l'isocyanate pur, du solvant pur ainsi qu'une partie de résidu, caractérisé en ce que le résidu obtenu à partir du processus de distillation est amené en continu dans un sécheur chauffé sous vide à arbre horizontal malaxant le produit avec 2 à 50% en poids d'hydrocarbures lourds, de préférence des bitumes, qui sont inertes dans les conditions de la distillation, la teneur en isocyanates encore présents dans le résidu étant extraite en continu par distillation à des températures de 160 à 280°C et une pression de 2 à 50 mbars et le résidu restant étant évacué en continu sous forme de granulat fluide, ne dégageant pas de poussière, le cas échéant refroidi et le cas échéant incinéré après broyage.

2. Procédé suivant la revendication 1, caractérisé en ce que le résidu est introduit dans le sécheur en plusieurs flux partiels, séparément des hydrocarbures.

3. Procédé suivant la revendication 1, caractérisé en ce qu'une partie du résidu est introduite dans le sécheur mélangée aux hydrocarbures et en ce que le flux restant est introduit dans le sécheur en un ou plusieurs flux partiels.

4. Utilisation d'un dispositif constitué d'un sécheur chauffé sous vide fonctionnant en continu, à arbre horizontal, malaxant le produit et de plusieurs amenées de produit réparties sur la longueur du sécheur, pour enlever les isocyanates de résidus de la distillation d'isocyanates.
